Europäisches Patentamt

European Patent Office

Office européen des brevets

(11) Publication number: **0 504 763 A1**

# EUROPEAN PATENT APPLICATION

(21) Application number: **92104436.8**

(22) Date of filing: **14.03.92**

(51) Int. Cl.5: **C12N 1/20, C12Q 1/24**

(30) Priority: **19.03.91 US 672206**

(43) Date of publication of application:
**23.09.92 Bulletin 92/39**

(84) Designated Contracting States:
**AT BE CH DE DK ES FR GB GR IT LI LU NL SE**

(71) Applicant: **Becton Dickinson and Company**
**One Becton Drive**
**Franklin Lakes New Jersey 07417-1880(US)**

(72) Inventor: **Evans, George L.**
**10301-L Malcolm Circle**
**Cockeysville, Maryland 21030(US)**
Inventor: **Marsik, Frederick J.**
**6 Keesey Road**
**New Freedom, Pennsylvania 17349(US)**
Inventor: **Eisenberg, Eli**
**54 Burla Street**
**Tel-Aviv(IL)**

(74) Representative: **von Kreisler, Alek,**
**Dipl.-Chem. et al**
**Patentanwälte Von Kreisler-Selting-Werner,**
**Deichmannhaus am Hauptbahnhof**
**W-5000 Köln 1(DE)**

(54) Ionizing irradiation sterilizable culture medium suitable for use in environmental sampling devices.

(57) A culture medium suitable for use in environmental sampling devices, which medium can be sterilized by ionizing radiation, is disclosed. The medium comprises a Soybean Casein digest agar, containing agents to neutralize disinfectants, which is modified by the addition of agar and yeast extract. The resultant medium can be sterilized by ionizing radiation without deleterious effects.

EP 0 504 763 A1

## BACKGROUND OF THE INVENTION

The use of culture media for the cultivation of microorganisms is known in the art. A wide assortment of media is known, and the particular media used can be customized to determine whether microorganisms can or cannot grow in a particular environment. Culture media are ordinarily designed to provide all of the nutrients which the microorganisms will utilize during growth, and can be in the forms of liquids, commonly referred to as broths, or solids, commonly referred to as gels. Solid media are of particular utility because they can be used to separate mixtures of microorganisms.

In normal use, a culture medium is sterilized (to kill microbial contamination) and subsequently put into sterile plates or containers, which are then stored in a germ free environment to assure that no subsequent microbial contamination occurs. Sterilization is highly important, as it is essential to remove all competing organisms from the media to assure that any growth observed will be only from the organisms which are intentionally placed on the media surface.

The type of sterilization used is mainly a matter of choice, and also of resources. While sterilization by heat (autoclaving) is used in many situations, not all media are directly amenable to such treatment. Specifically, high temperatures often will denature many of the proteins and other compounds present in media, rendering it useless for microbial growth.

The other sterilization technique used in the art is to expose the media to ionizing radiation to kill any microbial contaminant which may be present. This is accomplished by means of first preparing the culture medium, dispensing it into an appropriate container (e.g. a plate), and exposing the packaged medium to ionizing radiation of suitable intensity and duration to obtain a sterile package of the medium. The package, thus sterilized, can be stored for an extended period of time without ill effects.

While such sterilization has been used in a large number of applications, including sterilization of medical instruments, it is not as widely used for the sterilization of media. This is primarily due to the fact that the ionizing radiation fosters the formation of decomposition products in the media which may be deleterious to microbial growth. Further, in many cases the exposure to ionizing radiation causes deterioration of the agar component of solid media, rendering such media too soft for their intended applications. For this reason, autoclaving of such media has been the sterilization method of choice.

Eisenberg et al., in United States Patent No. 4,071,412 have described a method for producing media amenable to sterilization by radiation, by the insertion of compositions called "radio protectors", to prevent the degradation of products to form toxic substances. Specific examples of these radio protectors include indicators, vitamins, and enzymes, all of which will prevent the formation of certain toxic products during the irradiation. The patentees report a large increase in the stability of a number of specific media by the addition of said radio protectors.

However, the use of such radio protectors is not amenable to all media. For example, media used in environmental sampling devices such as contact plates (e.g. RODAC® plates) must remain solid and resilient to function in its intended manner. Contact plates are utilized to obtain samples of microbial contamination at various points within a room, process line, or other environment. In their use, the cover of the contact plate is removed, and the surface of the medium is pressed against the surface being sampled. The plate is then removed from that surface, re-covered, and incubated for an appropriate time, after which it is visually inspected for the appearance of colonies, which is indicative of microbial contamination. The colonies can be subsequently examined, counted, identified, etc. Contact plates can also be used to sample air for microbial contamination, using special devices designed for this application.

Unfortunately, when such plates are exposed to ionizing radiation, the integrity of the gel is compromised and the surface becomes too soft. This loss of gel strength, makes the media useless for contact applications.

Further, such plates often contain substances to neutralize disinfectants commonly found upon surfaces sampled. It is essential to neutralize these disinfectants, as their presence could inhibit microbial growth. Such compounds often interfere with the action of enzymes such as those used in the '412 patent, making such methods ineffective with contact plates.

There exists a real need, therefore, for environmental sampling media which can be sterilized by ionizing radiation, yet which will maintain their gel strength and not produce toxic substances which would interfere with microbial growth.

## SUMMARY OF INVENTION

It is therefore an object of this invention to present media suitable for use in environmental sampling devices which can be sterilized by ionizing radiation. It is further an object of this invention, to present a process for preparing sterile culture media in unit dosage form, for use in contact plates.

The above and related objects are achieved by the media of this invention. These media comprise a soybean casein digest agar (such as Trypticase® Soy Agar marketed by Becton Dickinson Microbiology Systems) containing additives which neutralize disinfectants (preferably lecithin and polysorbate 80. An agar of this type is marketed by Becton Dickinson as Trypticase® Soy Agar with Lecithin and Polysorbate 80. It has been found when these media are modified by the addition of yeast extract, agar and adjusting the pH to 7.4, the resultant media can be sterilized by ionizing radiation and still be capable of sustaining growth and retaining satisfactory gel strength. It is further anticipated that other media ordinarily used in environmental sampling devices such as Letheen agar and Standard Methods agar containing Lecithin and Polysorbate 80, can be rendered stable by the same modifications.

Such media, when sterilized by ionizing radiation, have been found to remain stable for storage periods of at least 4 months, supporting microbial growth as well as fresh media, and media sterilized by autoclaving after such storage.

## DETAILED DESCRIPTION OF THE INVENTION

The media of this invention comprise modified soybean casein digest agar which contains additives for neutralization of disinfectants (preferably lecithin and polysorbate 80). The modifications are achieved by the addition of yeast extract, agar, and adjustment of the pH to 7.4.

The yeast extract added is a conventional yeast extract, and is added at a treatment level of 3-10 grams per liter, preferably 4-8 grams per liter, and more preferably 5 grams per liter. The agar (preferably a bacteriologic grade agar) is ordinarily (but not essentially) added at the same treatment level, and is preferably added at 5 grams per liter. The pH is preferably adjusted to 7.4, but has been found that pH's of 7.2-7.6 give acceptable results. Adjustment of the pH is preferably made by using 0.1N NaOH, or by the addition of $Na_2CO_3$. A particular advantage of $Na_2CO_3$ is that it can be blended with the dry medium prior to gel preparation, obviating the need for a separate neutralization procedure.

These modified media can be sterilized by autoclaving, but can also be terminally sterilized by irradiation with ionizing radiation. The preferable ionizing radiation used is gamma radiation, but is also possible to use other types of ionizing radiation such as X-rays and electron beams of suitable intensity, the only criterion being that the radiation be of a suitable intensity to achieve sterilization within a reasonable amount of time. Preferred sources of gamma irradiation are cesium ($Cs^{137}$) and cobalt ($Co^{60}$).

In normal use, the prepared medium is poured into the environmental sampling device, preferably a contact plate, which is closed and sealed in a package. The package is then exposed to the ionizing radiation. In this way, a clean sterile package containing a single device, or a plurality of devices, is obtained and the package can be stored until needed for use. While room temperature storage is possible, it is preferred that the devices be stored at 2-8°C, to maximize the shelf life. It has been found that media, thus sterilized, can be stored at this temperature for approximately 4 months, without any deleterious effects.

The devices, containing the sterilized media, can then be utilized in the desired application. In a preferred embodiment of this invention, the device is a contact plate. In normal use, the plate cover is removed, and the surface of the medium is placed against the surface or exposed to the environment to be sampled. Immediately after removing the plate, the cover is replaced, and the system is incubated to determine if microbial growth occurs. It has been found that the plates of this invention do not exhibit significantly less growth for most microorganisms compared with autoclaved media. Furthermore, the media of this invention will neutralize disinfectants ordinarily found on sampled surfaces. Thus, the plates of this invention can be used in virtually all applications where contact plates would normally be used.

## EXAMPLES

The following examples demonstrate certain preferred embodiments of the instant invention, but are intended to be illustrative of all embodiments.

### EXAMPLE 1 Comparative Effects of Additives

To assess the effect of different additives to the media, and different quantities of these additives, a series of experiments were run utilizing Becton Dickinson Trypticase® soy agar containing Lecithin and Polysorbate 80 (TSA-LP), and either catalase, or yeast extract and bacteriological grade agar as additional additives. In each experiment, dehydrated TSA-LP was prepared according to label instructions and modified by the addition of desired additives. The pH was then adjusted to 7.4 with 0.1N NaOH.

The media were placed in sterile plates which were, as applicable, exposed to the desired dose of gamma radiation from a $Co^{60}$ source, and inoculated with Staphylococcus aureus ATCC 25923 or Streptococcus pyogenes ATCC 19615 using 0.1 ml aliquots having a viable count expected to produce 30-300 CFU (colony forming units). Duplicate plates were inoculated for each determination and the results are reported as the average CFU/plate and the mean colony diameter in mm, after a 10-24 hour incubation at 35°C. The results are presented in Table I.

TABLE I

| Results of Comparative Additive Experiments | | | | | | |
|---|---|---|---|---|---|---|
| Additive | | | Results | | | |
| Rad. Dose (Mrad) | Agar[a] (g/L) | Other | S. Aureus | | S. pyogenes | |
| | | | CFU | Size (mm) | CFU | Size (mm) |
| None | 0 | None | 192 | 2.1 | 95 | 1.4 |
| | 0 | Catalase[b] | 232 | 2.2 | 77 | 1.2 |
| | 0 | Yeast Extract[c] | 212 | 2.3 | 82 | 2.8 |
| 0.8 | 3 | None | 173 | 1.6 | 96 | 1.1 |
| | 3 | Catalase | 190 | 1.8 | 85 | 0.7 |
| | 3 | Yeast Extract | 170 | 1.8 | 69 | 2.7 |
| 3.2 | 6 | None | 179 | 1.0 | 30 | <0.3 |
| | 6 | Catalase | 225 | 1.3 | 100 | 0.4 |
| | 6 | Yeast Extract | 196 | 1.4 | 95 | 1.7 |

Notes
[a] Initial agar concentration (without additives) is 15 g/L
[b] Catalase added to a 100 mg/L concentration
[c] Yeast extract added to a 5 g/L concentration

As shown, at zero and low (0.8 Mrad) radiation doses, the CFU is not significantly enhanced by any additive. However, at a dose of 3.2 Mrad, both yeast extract and catalase exhibit enhanced recovery (CFU) compared with agar alone. Further, in all three series, the media containing the yeast extract exhibited enhanced growth, as evidenced by the larger mean colony size of the S. pyogenes.

Example 2 Use of Different Bacterial Strains

In this series of experiments, duplicate plates of plain TSA-LP and TSA-LP modified by the addition of 5 g/L each of additional agar and yeast extract and adjusting the pH to 7.4 with 0.1N NaOH (Modified TSA-LP) were inoculated with microorganisms as in Example 1. The results are presented in Table II.

TABLE II

| Organism | MODIFIED TSA-LP | | | | | |
|---|---|---|---|---|---|---|
| | TSA-LP | | NON-IRRADIATED | | IRRADIATED[a] | |
| | CFU | Colony Size (mm) | CFU | Colony Size (mm) | CFU | Colony Size (mm) |
| Bacillus subtilis (vegetative) ATCC 6633 | 65 | 3.7 | 52 | 5.9 | 31 | 4.4 |
| Staphylococcus aureus ATCC 25923 | 55 | 1.9 | 57 | 1.9 | 47 | 1.8 |
| Enterococcus faecalis ATCC 29212 | 65 | 1.1 | 65 | 1.3 | 69 | 1.3 |
| Salmonella typhimurium ATCC 13311 | 103 | 2.5 | 98 | 2.8 | 105 | 2.6 |
| Micrococcus luteus ATCC 9341 | 48 | 0.8 | 47 | 0.8 | 61 | 0.9 |
| Candida albicans BDMS 001 | 7 | 2.3 | 5 | 1.9 | 6 | 1.9 |
| Streptococcus pyogenes ATCC 19615 | 69 | 0.7 | 71 | 1.2 | 66 | 1.0 |
| Pseudomonas aeruginosa ATCC 10145 | 140 | 2.2 | 127 | 2.1 | 122 | 1.9 |
| Aspergillus niger ATCC 16404 | growth | | growth | | growth | |

Notes

[a] 1.5 Mrad from a $Cs^{137}$ source

As shown, the recovery (CFU) and growth (size) was comparable for the irradiated and non-irradiated media, except for B. subtilis (vegetative) which showed a decrease. Thus, the Modified TSA-LP can be used to support growth of a wide variety of microorganisms.

A second series of experiments was conducted comparing the growth of these organisms on the same media stored for 4 months at 2-8°C, as compared with freshly prepared TSA-LP. The results are presented in Table III.

TABLE III

| Organism | Fresh TSA-LP CFU | Colony Size (mm) | TSA-LP CFU | Colony Size (mm) | STORED MEDIA[b] Modified TSA-LP NON-IRRADIATED CFU | Colony Size (mm) | IRRADIATED[a] CFU | Colony Size (mm) |
|---|---|---|---|---|---|---|---|---|
| Bacillus subtilis (vegetative) ATCC 6633 | 68 | 2.2 | 21 | 1.4 | 26 | 2.5 | 57 | 2.9 |
| Bacillus subtilis (spores) ATCC 6633 | 280 | 1.9 | 283 | 2.3 | 263 | 3.3 | 243 | 3.1 |
| Staphylococcus aureus ATCC 25923 | 64 | 1.8 | 67 | 1.6 | 72 | 1.8 | 65 | 1.6 |
| Pseudomona aeruginosa ATCC 10145 | 80 | 2.0 | 78 | 3.0 | 81 | 2.6 | 69 | 2.5 |
| Salmonella typhimurium ATCC 13311 | 80 | 2.9 | 80 | 2.4 | 76 | 2.5 | 88 | 2.5 |
| Streptococcus pyogenes ATCC 19615 | 70 | 0.8 | 77 | 0.6 | 86 | 1.0 | 80 | 1.2 |
| Candida albicans BDMS 001 | 425 | 0.9 | 335 | 0.7 | 375 | 0.8 | 322 | 0.7 |
| Enterococcus faecalis ATCC 29212 | 43 | 1.5 | 44 | 1.3 | 43 | 1.7 | 63 | 1.3 |
| Micrococcus luteus ATCC 9341 | 480 | 0.8 | 475 | 0.7 | 458 | 0.8 | 480 | 0.7 |
| Aspergillus niger ATCC 16404 | growth | | growth | | growth | | growth | |

Notes

<sup>a</sup> 1.5 Mrad from a Cs<sup>137</sup> source

<sup>b</sup> Stored 4 months at 2-8°C

As shown, the stored media exhibited comparable recovery and size, except for the vegetative cells of B. subtilis, which actually showed significantly enhanced recovery when irradiated.

Example 3 Neutralizing Efficiency

The neutralizing efficiency for quartenary ammonium compounds was determined using a modification of the Hoffmann and Phillips assay (Ann. N.Y. acacl Aci. 53, pp 59-65). Briefly, plates were inoculated with S. aureus ATCC 6538P, and paper discs (12mm in diam) moistened with 0.08 ml benzalkonium chloride solution, at concentrations of 8, 4, 2, 1, 0.5, or 0.25 mg/ml were then placed on the surface. Each plate contained three discs of different concentrations and each concentration was tested on three plates. After a 24 hour incubation at 25°C, the diameter of each inhibition zone was measured. Using linear regression analysis (the log concentration varies linearly with zone diameter), the concentration needed to produce no inhibition zone was calculated; this is the neutralizing efficiency in mg/ml. The results are summarized in Table IV.

TABLE IV

| Neutralizing Efficiency | | | |
|---|---|---|---|
| Media | Irradiated$^a$ | Fresh (mg/ml) | Stored$^b$ |
| TSA-LP | No | 0.255 | 0.234 |
| Modified TSA-LP | No | 0.253 | 0.262 |
| Modified TSA-LP | Yes | 0.264 | 0.281 |

Notes
$^a$ 1.5 Mrad from a Cs$^{137}$ source
$^b$ Stored 4 months at 2-8°C

As shown, all media exhibited similar results, indicating that the irradiated media sustain microbial growth as well as the non-irradiated media.

Example 4 Gel Strength

Gel Strength was determined by the measure of Stoloff (Fishery leaflet 306, U.S. Dept. of the Int.). The results are presented in Table V.

TABLE V

| Gel Strength | | | |
|---|---|---|---|
| Media | Irradiated$^a$ | Fresh | Stored$^b$ |
| TSA-LP | No | 569 g/cm$^2$ | 547 g/cm$^2$ |
| Modified TSA-LP | No | 808 g/cm$^2$ | 849 g/cm$^2$ |
| Modified TSA-LP | Yes | 618 g/cm$^2$ | 593 g/cm$^2$ |

Notes
$^a$ 1.5 Mrad from a Cs$^{137}$ source
$^b$ Stored 4 months at 2-8°C

As shown, the modified TSA-LP has a higher gel strength than the standard (fresh) media. Following irradiation, the gel strength of the modified media is comparable to the standard.

It is apparent that many modifications and variations of this invention as hereinabove set forth may be made without departing from the spirit and scope hereof. The specific embodiments described are given by way of example only and the invention is limited only by the terms of the appended claims.

**Claims**

1. In a microbial culture medium adapted for use in environmental sampling devices which medium comprises an agar medium and one or more additives for neutralization of disinfectants, the improvement comprising containing within said medium an effective amount of an agar and a yeast extract such that, when the pH is 7.2-7.6, the medium can be sterilized with ionizing radiation and maintain its ability to sustain microbial growth and gel strength.

2. The microbial culture medium of Claim 1 wherein the agar medium is a soybean casein digest agar.

3. The microbial culture medium of Claim 1 wherein the agar medium is a Letheen agar.

4. The microbial culture medium of Claim 1 wherein the agar medium is standard methods agar.

5. The medium of Claim 1, wherein the additives are lecithin and polysorbate 80.

6. The medium of Claim 1, wherein the agar and yeast extract are each present at a concentration level of 3-10 g/L.

7. The medium of Claim 1, wherein the agar is a bacteriological grade agar.

8. The medium of Claim 6, wherein the agar and yeast extract are each present in the agar at a concentration level of 5 g/L.

9. The medium of Claim 1, wherein the pH is adjusted by the addition of NaOH or $Na_2CO_3$.

10. The medium of Claim 1, wherein the pH is 7.4.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | BIOSIS PREVIEWS DATABASE,Philadelphia Abstract No. 78090274 ,Koller W." Recovery of test bacteria..." & KRANKENHAUSHYG.ARBEITSHYG. PRAEV.MED 179 2 1984 112-124 | 1 | C12N1/20 C12Q1/24 |
| A | | 2 | |
| Y | CA-A-1 061 730 (MARMEL ,M. ET AL.) | 1 | |
| A | | 6,8,10 | |
| | * example 5 * | | |
| A | DE-A-2 519 685 (STATE OF ISRAEL VERTRETEN DURCH ATOMIC ENERGY COMMISSION ET AL.) * the whole document * | 1 | |
| D | & US-A-4071412 | | |

| | | | TECHNICAL FIELDS SEARCHED (Int. Cl.5) |
|---|---|---|---|
| | | | C12N C12Q |

The present search report has been drawn up for all claims

| Place of search | Date of completion of the search | Examiner |
|---|---|---|
| BERLIN | 26 JUNE 1992 | GURDJIAN D. |